# EUROPEAN PATENT APPLICATION

(11) **EP 4 752 129 A1**
(43) Date of publication of application: **03.06.2026**
(21) Application number: 24844800.3
(22) Date of filing: 24.07.2024
(51) Int. Cl.: C07C 67/31, C07C 69/675, C07C 231/02, C07C 235/06

(54) **METHOD FOR PREPARING METHYL 3-METHOXYPROPIONATE AND 3-METHOXY-N,N-DIMETHYL PROPIONAMIDE**

(30) Priority: 25.07.2023 CN 202310923609
(71) Applicant: Guangzhou Tinci Materials Technology Co., Ltd, Guangzhou, Guangdong 510700 (CN); Jiujiang Tinci Materials Technology Co., Ltd., Jiujiang, Jiangxi 332500 (CN)
(72) Inventor: WANG, Kai, Guangzhou, Guangdong 510700 (CN); FAN, Weizhen, Guangzhou, Guangdong 510700 (CN); SHI, Litao, Guangzhou, Guangdong 510700 (CN)
(74) Representative: Pfenning, Meinig & Partner mbB
(86) International application number: PCT/CN2024/107248
(87) International publication number: WO 2025/021109

(57) **Abstract**

A method for preparing a 3-methoxypropionate. The method includes the following steps: step 1, taking an acrylate and methanol as raw materials, and carrying out a Michael addition reaction in the presence of a basic catalyst, so as to obtain a first mixed solution including methyl 3-methoxypropionate; and step 2, treating the first mixed solution by means of distillation, and collecting fractions in a preset temperature section, so as to obtain methyl 3-methoxypropionate, where the molar ratio of the basic catalyst to the acrylate is less than 0.6:100. By means of the method, methyl 3-methoxypropionate can be directly obtained without neutralizing the basic catalyst. In addition, further provided is a method for preparing 3-methoxy-N,N-dimethyl propionamide.

## Description

The present application claims the priority to Chinese Patent Application No. CN202310923609.5, titled "METHOD FOR PREPARING METHYL 3-METHOXYPROPIONATE AND 3-METHOXY-N,N-DIMETHYLPROPIONAMIDE", filed before the China National Intellectual Property Administration on July 25, 2023, which is incorporated herein by reference in its entirety.

### TECHNICAL FIELD

The present application belongs to the field of new energy, and specifically relates to a method for preparing methyl 3-methoxypropionate and a method for preparing 3-methoxy-N,N-dimethylpropionamide.

### BACKGROUND

3-Methoxy-N,N-dimethylpropionamide is miscible with various solvents. It can dissolve polymer polyamide to a high degree, and has characteristics such as high solubility, high permeability, and low viscosity. It is non-irritating to the skin, safe, and environmentally friendly. It has wide applications in coatings, inks, dyes, and semiconductor cleaning. Furthermore, it can be used as a solvent in the preparation of lithium-ion battery separator base films, separator coatings, polyimide separator preparation, and non-woven separators, etc. Its performance is comparable to that of acetone, and environmental issues, cost problems, and health risks are all resolved.

Methyl 3-methoxypropionate is an intermediate in the synthesis of 3-methoxy-N,N-dimethylpropionamide.

Regarding the synthesis of methyl 3-methoxypropionate, the following literature can be referred to:
CN1258518C (basic catalyst method), CN112479874A (basic catalyst method, tertiary/quaternary amine), CN113754537A (basic catalyst method, tertiary/quaternary amine), CN101616889B (basic catalyst method), CN104203905A (WO2013153754A1, basic catalyst method), CN102245561A (WO2010067589A1, basic catalyst method), CN106883136A (other methods), CN106966923B (other methods), CN112961051A (basic catalyst method), CN105801417A (basic catalyst method), CN105037151A (supported catalyst method), CN116116402A (supported catalyst method).

In terms of preparation routes, they can be divided into basic catalyst methods and supported catalyst methods.

The basic catalytic method is inferior to the supported catalytic method in many aspects, with the most criticized aspect being: after the reaction of the basic catalytic method, neutralization is mostly required to obtain methyl 3-methoxypropionate.

Specifically, refer to Examples 1 to 4 of Prior Art 1: CN101616889B PROCESS FOR PRODUCING B-ALKOXYPROPIONAMIDE, where the basic catalyst is neutralized with acid after the reaction. However, this brings a significant industrial operational burden, seriously affecting production efficiency. Meanwhile, neutralization salts are generated. These neutralization salts are not only waste but also require subsequent filtration operations before distillation. In summary, neutralizing the basic catalyst with acid is the least suitable treatment method in this project.

To avoid neutralization, Prior Art 1 creatively uses a production method without neutralization. Specifically, refer to Example 5 and Example 8 thereof:

### Example 5

To a 200 ml four-necked flask equipped with a stirrer, a dropping funnel, a Dimroth condenser and a thermometer, 41.33 g (1.29 mol) of methanol and 1.30 g (0.024 mol) of sodium methoxide as the basic catalyst were added.

74.04 g (0.86 mol) of methyl acrylate was weighed and gradually added dropwise from the dropping funnel. In this case, since heat is generated when a small amount of methyl acrylate is added dropwise, the reaction temperature was controlled to 40°C by cooling with ice water. After the dropwise addition for 1 hour, the reaction was conducted for 1 hour.

Thereafter, 0.39 g (0.007 mol) of sodium methoxide was added to the flask at room temperature (about 20°C). Then, a dimethylamine gas was blown by a circulating system. The reaction was conducted for 4 hours under atmospheric pressure. Upon blowing dimethylamine, the reaction temperature reached a maximum of 50°C.

After the reaction, the liquid was taken out of the flask and neutralized by adding 2.46 g (0.024 mol) of phosphoric acid (85% aqueous solution).

### Example 8

A reaction was conducted in the same manner as in Example 5, except that the amount of methanol was changed to 27.55 g (0.86 mol) and sodium methoxide was not added at the time of the amidation reaction, whereby β-methoxy-N, N-dimethylpropionamide was obtained.

Although Example 8 did not use an additionally added catalyst during the amidation reaction, in fact, the catalyst from the Michael addition reaction was retained in the amidation reaction.

From the above cases, the following conclusion can basically be drawn:
During the amidation reaction process, a catalyst is generally required. If no catalyst is used, the conversion ratio and selectivity are poor, as in Comparative Example 1 of Prior Art 1, as follows:

### Comparative Example 1

To a 200 ml four-necked flask under a dry air stream, 70.88 g (0.6 mol) of methyl β-methoxypropionate and 36.8 g (0.4 mol) of glycerin serving as both catalyst and solvent were added. The resultant was heated to 80°C under atmospheric pressure and reacted for 20 hours while continuously blowing a dimethylamine gas.

The conversion ratio of methyl β-methoxypropionate was 46 wt%, and the selectivity for β-methoxy-N, N-dimethylpropionamide was 51 wt%.

Under such conditions, if methanol were retained as in Example 5 and Example 8, the situation would become worse, because methanol would dilute the reaction system, leading to a slower reaction rate. Meanwhile, an increased amount of methanol would significantly increase the required amount of dimethylamine. Most critically, the final process required neutralization, and once neutralized, a filtration step would still be added.

The practical challenges are as follows:
1. If the method of Example 5 of Prior Art 1 is adopted, the reaction speed is slow and the yield is low;
2. If the method of Example 1 of Prior Art 1 is adopted, it is necessary to neutralize and then separate to obtain the 3-methoxypropionate.

In preliminary experiments, it was found that if the catalyst was not neutralized and the light components were directly distilled off, both the yield and selectivity dropped sharply, making it essentially impossible to obtain the product. The amount of by-products is an indicator of selectivity; the more by-products, the lower the selectivity; the fewer by-products, the higher the selectivity.

Therefore, the problems addressed by this project are:
1. How to directly obtain methyl 3-methoxypropionate with high yield and high selectivity without neutralizing the catalyst in the Michael addition reaction catalyzed by a basic catalyst;
2. How to avoid neutralization and filtration throughout the entire production process of preparing 3-methoxy-N,N-dimethylpropionamide via the basic catalytic method.

### SUMMARY

In view of the deficiencies of the prior art, an object of the present application is to provide a method for preparing methyl 3-methoxypropionate, which does not require neutralization of the basic catalyst and enables direct recovery of methyl 3-methoxypropionate.

Meanwhile, the present application also provides a method for preparing 3-methoxy-N,N-dimethylpropionamide.

In the present application, "less than" and "greater than" do not include the number itself, and "not less than", "not higher than", "not more than", "not greater than", and "not smaller than" include the number itself.

To achieve this object, the present application adopts the following technical solution: a method for preparing a 3-methoxypropionate, including the following steps:
step 1: carrying out a Michael addition reaction using an acrylate and methanol as raw materials in the presence of a basic catalyst to obtain a first mixed solution containing methyl 3-methoxypropionate; and
step 2: treating the first mixed solution by distillation, and collecting distillate fractions in a preset temperature range to obtain methyl 3-methoxypropionate;
where a molar ratio of the basic catalyst to the acrylate is less than or equal to 0.6:100, preferably, the molar ratio of the acrylate to the catalyst ranges from 100:0.2 to 100:0.6;
a chemical formula of the acrylate is: 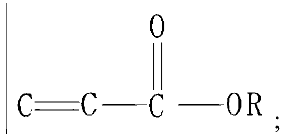 and
R is an alkyl group having 1 to 3 carbon atoms.

It should be noted:
The preset temperature range in the present application is related to a pressure of the distillation; the lower the pressure, the lower the distillation temperature, for example:
when the vacuum degree is -0.06 MPa, the preset temperature range is 112°C to 116°C;
when the vacuum degree is -0.07 MPa, the preset temperature range is 102°C to 107°C;
when the vacuum degree is -0.08 MPa, the preset temperature range is 92°C to 96°C;
when the vacuum degree is -0.09 MPa, the preset temperature range is 78°C to 82°C;
when the vacuum degree is -0.095 MPa, the preset temperature is 68°C to 73°C.

Therefore, the preset temperature range can be controlled from 65°C to 120°C, preferably from 68°C to 116°C, depending on the pressure.

In the present application, the distillate fractions in the preset temperature range obtained in the step 2 are, in most cases, not pure methyl 3-methoxypropionate. From the perspective of rectification, the taller the column and the greater the number of plates, the higher the purity of the distillate. However, this also means higher investment costs and energy consumption. In the step 2 of the present application, the vast majority of the obtained distillate fractions is methyl 3-methoxypropionate, with a small amount of methanol. Methanol will not have a decisive impact on the subsequent amidation reaction, except for a slight weakening effect on the reaction rate and conversion ratio. Therefore, the presence of methanol is permissible in the distillate fractions of the step 2 of the present application. Preferably, the distillate fractions contain not less than 90 wt% of methyl 3-methoxypropionate; more preferably, the distillate fractions contain not less than 95 wt% of methyl 3-methoxypropionate; more preferably, the distillate fractions contain not less than 97 wt% of methyl 3-methoxypropionate; more preferably, the distillate fractions contain not less than 99 wt% of methyl 3-methoxypropionate; more preferably, the distillate fractions contain not less than 99.5 wt% of methyl 3-methoxypropionate; more preferably, the distillate fractions contain not less than 99.8 wt% of methyl 3-methoxypropionate.

In the present application, the statement "a molar ratio of the basic catalyst to the acrylate is less than or equal to 0.6:100" means that the basic catalyst must be used and the ratio must be less than or equal to this ratio.

In some embodiments of the present application, the molar ratio of the basic catalyst to the acrylate includes but is not limited to 0.6:100, 0.55:100, 0.5:100, 0.45:100, 0.4:100, 0.35:100, 0.3:100, 0.25:100, 0.2:100, 0.1:100, 0.05:100, or any range less than or equal to 0.6:100.

The basic catalyst of the present application does not include supported catalysts bearing a basic catalyst. The basic catalyst of the present application is selected from alkali (earth) metal alkoxides, such as sodium alkoxide; alkali (earth) metal oxides, such as sodium oxide, potassium oxide; alkali (earth) metal hydroxides, such as sodium hydroxide, potassium hydroxide; alkali (earth) metal carbonates, such as sodium carbonate, potassium carbonate; as well as some quaternary ammonium salt and organic base-type basic catalysts.

The present application precisely controls the amount of catalyst used in the Michael addition reaction. At this amount of catalyst, it can be ensured that the product does not decompose or polymerize during distillation. Once this ratio is exceeded, it leads to further reaction of the product 3-methoxy-N,N-dimethylpropionamide with dimethylamine to generate the by-product 3-dimethylamino-N,N-dimethylpropionamide, and decomposition of 3-methoxy-N,N-dimethylpropionamide to generate methyl 3-(dimethylamino)acrylate.

In the separation operation of the step 2, after removing methanol, the product is collected in a specific temperature range. The heavy component generated from rectification can be recycled to the step 1, thereby achieving the reuse of raw materials and catalyst. If the ratio of the present application is exceeded, excessive by-products are generated, rendering it unsuitable for recycling.

Of course, the molar ratio of the basic catalyst to the acrylate is also not recommended to be too small. Although too small a ratio can avoid by-product formation, it is not beneficial for the conversion ratio. Excessive recycling of raw materials would significantly increase production costs.

In the above method for preparing a 3-methoxypropionate, a molar amount of methanol is not less than 1.5 times a molar amount of the acrylate.

In the above method for preparing a 3-methoxypropionate, a molar ratio of the acrylate to methanol ranges from 1:1.5 to 1:3.

In some embodiments of the present application, the molar ratio of the acrylate to methanol includes but is not limited to 1:1.5, 1:1.6, 1:1.7, 1:1.8, 1:1.9, 1:2.0, 1:2.1, 1:2.2, 1:2.3, 1:2.4, 1:2.5, 1:2.6, 1:2.7, 1:2.8, 1:2.9, 1:3.0, or any range formed between any two of these values.

In the above method for preparing a 3-methoxypropionate, in the step 2, the first mixed solution is treated by distillation under reduced pressure, distillate fractions in the preset temperature range are collected to obtain methyl 3-methoxypropionate;
a pressure of the distillation ranges from -0.06 MPa to -0.1 MPa, preferably from -0.06 MPa to -0.095 MPa; and
the preset temperature is 65°C to 120°C, preferably 68°C to 116°C.

The pressure values shown in the present application are gauge pressures, not absolute pressures. That is, atmospheric pressure is taken as 0 MPa (absolute pressure 101 kPa). Values below atmospheric pressure represent the pressures indicated in the present application. For example, -0.095 MPa means 95 kPa below atmospheric pressure, with an absolute pressure of about 6 kPa. In some geographical environments, such as plateau environments where the atmospheric absolute pressure ranges from 72 kPa to 96 kPa, the gauge pressure values shown in the present application cannot be directly equivalently substituted. The above pressures should be converted to absolute pressures. That is, when converting the pressure units of the present application, they should be calculated based on the atmospheric pressure at sea level to obtain the absolute pressure.

As mentioned above, the selection of the distillation temperature is related to the pressure. Therefore, for those skilled in the art, when the distillation pressure ranges from -0.06 MPa to -0.1 MPa, as the vacuum degree gradually increases, the distillation temperature will gradually decrease from 120°C to 65°C. Thus, the distillation pressure calculated based on vacuum degree and the temperature have a negative correlation.

In the above method for preparing a 3-methoxypropionate, in the step 2, methanol is first removed by distillation under reduced pressure. Then the temperature is raised, and the distillate fractions in the preset temperature range are collected by distillation under reduced pressure.

The "removing methanol" mentioned herein does not mean removing 100% of the methanol from the system. As is well known, in rectification operations, it is difficult to completely remove a substance from the substrate. In the present application, even if there is a small or trace amount of methanol remaining in the substrate after methanol removal, it will not affect subsequent operations.

In the above method for preparing a 3-methoxypropionate, when the pressure of the distillation ranges from -0.09 MPa to -0.1 MPa, rectification is performed using a rectification column with stepwise temperature increase; within the range of 50°C to 80°C, stepwise temperature increase operations are performed to achieve the distillation of methanol; then the temperature is raised to 90°C and gradually increased to 120°C, and distillate fractions at 68°C to 73°C are collected.

The operation of performing stepwise temperature increase within the range of 50°C to 80°C to achieve the distillation of methanol can be performed as follows: distillation is performed at 50°C, and methanol is collected as the distillate; then the temperature is raised to 60°C for distillation, and methanol is collected; then the temperature is raised to 70°C for distillation, and methanol is collected; then the temperature is raised to 80°C for distillation, and methanol is collected;
the operation of performing stepwise temperature increase within the range of 50°C to 80°C to achieve the distillation of methanol can also be performed as follows: distillation is performed at 50°C, and methanol is collected; then the temperature is raised to 70°C for distillation, and methanol is collected; then the temperature is raised to 80°C for distillation, and methanol is collected;
the operation of performing stepwise temperature increase within the range of 50°C to 80°C to achieve the distillation of methanol can also be performed as follows: distillation is performed at 50°C, and methanol is collected; then the temperature is raised to 80°C for distillation, and methanol is collected;
the operation of raising the temperature to 90°C and gradually increasing it to 120°C, and collecting distillate fractions from 68°C to 73°C can be performed as follows: the temperature is raised to 90°C, and distillate fractions at 68°C to 73°C are collected; then the temperature is raised to 100°C, and distillate fractions at 68°C to 73°C are collected; then the temperature is raised to 110°C, and distillate fractions at 68°C to 73°C are collected; then the temperature is raised to 120°C, and distillate fractions at 68°C to 73°C are collected;
the operation of raising the temperature to 90°C and gradually increasing it to 120°C, and collecting distillate fractions at 68°C to 73°C can also be performed as follows: the temperature is raised to 90°C, and distillate fractions at 68°C to 73°C are collected; then the temperature is raised to 100°C, and distillate fractions at 68°C to 73°C are collected; then the temperature is raised to 120°C, and distillate fractions at 68°C to 73°C are collected;
the operation of raising the temperature to 90°C and gradually increasing it to 120°C, and collecting distillate fractions at 68°C to 73°C can also be performed as follows: the temperature is raised to 90°C, and distillate fractions at 68°C to 73°C are collected; then the temperature is raised to 120°C, and distillate fractions at 68°C to 73°C are collected;
in the above method for preparing a 3-methoxypropionate, a bottom product from the rectification column is a first heavy component containing the basic catalyst and 3-methoxypropionate; the first heavy component is recycled to the step 1.

Because the present application precisely controls the amount of catalyst, even when the temperature is raised to 120°C, it has been detected that the first heavy component in the column bottom does not significantly contain other by-products. This provides a basis for the recycling of the bottom product.

In the above method for preparing a 3-methoxypropionate, R is methyl, ethyl, or propyl.

In the above method for preparing a 3-methoxypropionate, the basic catalyst is one or more of sodium methoxide, sodium ethoxide, sodium tert-butoxide, potassium tert-butoxide, sodium hydride, sodium amide, sodium hydroxide, potassium hydroxide, sodium oxide, potassium oxide, sodium carbonate, potassium carbonate, sodium bicarbonate, potassium bicarbonate, potassium fluoride, and choline.

In the above method for preparing a 3-methoxypropionate, a reaction temperature of the step 1 ranges from 40°C to 60°C, and a reaction time ranges from 1 hour to 5 hours.

Preferably, the reaction temperature of the step 1 ranges from 45°C to 55°C, and the reaction time ranges from 1.5 hours to 3.5 hours.

In some specific embodiments of the present application, the reaction temperature of the step 1 is 40°C, 45°C, 50°C, 55°C, or 60°C.

In some specific embodiments of the present application, the reaction time of the step 1 is 1 hour, 1.5 hours, 2 hours, 2.5 hours, 3 hours, 3.5 hours, 4 hours, 4.5 hours, or 5 hours. A longer time is also feasible but is not beneficial for cost.

Meanwhile, the present application also discloses a method for preparing 3-methoxy-N,N-dimethylpropionamide, including the following steps:
step 3: carrying out an amidation reaction using the distillate fractions in the preset temperature range as described above, a polyhydric alcohol, and dimethylamine as raw materials to obtain a second mixed solution containing 3-methoxy-N,N-dimethylpropionamide.

In the above method for preparing 3-methoxy-N,N-dimethylpropionamide, after the step 3, it further includes:
step 4: treating the second mixed solution to remove dimethylamine and methanol, so as to obtain a third mixed solution; and
step 5: subjecting the third mixed solution to rectification with stepwise temperature increase under reduced pressure, to obtain a 3-methoxypropionate and 3-methoxy-N,N-dimethylpropionamide respectively in different temperature ranges, and a bottom product is the polyhydric alcohol;
preferably, the 3-methoxypropionate and the polyhydric alcohol obtained in the step 5 are recycled to the step 3.

The "rectification with stepwise temperature increase under reduced pressure" in the present application refers to, under conditions below atmospheric pressure, gradually increasing the heating temperature at the bottom of the rectification column to achieve the output of distillate fractions at different temperature ranges, thereby obtaining substances with different boiling points;
in the above method for preparing 3-methoxy-N,N-dimethylpropionamide, a reaction temperature of the step 3 is not more than 60°C, preferably not more than 50°C, more preferably not more than 40°C.

In some embodiments of the present application, the reaction temperature of the step 3 is 10°C, 20°C, 30°C, 40°C, 50°C, or 60°C;
in the above method for preparing 3-methoxy-N,N-dimethylpropionamide, the reaction temperature of the step 3 ranges from 30°C to 50°C, preferably from 30°C to 40°C.

In some preferred embodiments of the present application, the reaction temperature of the step 3 is 30°C, 32°C, 34°C, 36°C, 38°C, 40°C, 42°C, 44°C, 46°C, 48°C, or 50°C.

In the above method for preparing 3-methoxy-N,N-dimethylpropionamide, a molar amount of the 3-methoxypropionate, a molar amount of dimethylamine, and a molar amount of the polyhydric alcohol are in a ratio ranging from 1:(1.2 to 3):(0.5 to 2), preferably, the molar amount of the 3-methoxypropionate, the molar amount of dimethylamine, and the molar amount of the polyhydric alcohol are in a ratio ranging from 1:(2 to 3):(0.5 to 2).

In some embodiments of the present application, a ratio of the molar amount of the 3-methoxypropionate to the molar amount of dimethylamine is 1:1.2, 1:1.4, 1:1.6, 1:1.8, 1:2.0, 1:2.2, 1:2.4, 1:2.6, 1:2.8, or 1:3.0.

In some embodiments of the present application, a ratio of the molar amount of the 3-methoxypropionate to the molar amount of the polyhydric alcohol is 1:0.5, 1:0.7, 1:0.9, 1:1.0, 1:1.2, 1:1.4, 1:1.6, 1:1.8, or 1:2.0.

In the above method for preparing 3-methoxy-N,N-dimethylpropionamide, a reaction time of the step 3 is not less than 4 hours, preferably, the reaction time of the step 3 ranges from 4 hours to 15 hours.

In some embodiments of the present application, the reaction time of the step 3 can be 4 hours, 5 hours, 6 hours, 7 hours, 8 hours, 9 hours, 10 hours, 11 hours, 12 hours, 13 hours, 14 hours, or 15 hours.

In the above method for preparing 3-methoxy-N,N-dimethylpropionamide, the polyhydric alcohol is one or more of glycerol, ethylene glycol, pentaerythritol, 1,2-propanediol, 1,4-butanediol, and 1,6-hexanediol.

Compared to the prior art, the present application has the following beneficial effects:
(1) The present application precisely controls the amount of catalyst used in the Michael addition reaction. At this amount of catalyst, it can be ensured that the product does not decompose or polymerize during distillation.
(2) By optimizing the amount of dimethylamine and temperature control, the present application achieves high conversion ratio and high selectivity under catalyst-free conditions.

### BRIEF DESCRIPTION OF THE DRAWINGS

FIG. 1 is the proton nuclear magnetic resonance (H-NMR) spectrum of the product of Example 1;
FIG. 2 is the carbon nuclear magnetic resonance (C-NMR) spectrum of the product of Example 1;
FIG. 3 is the proton nuclear magnetic resonance (H-NMR) spectrum of the product of Example 21;
FIG. 4 is the carbon nuclear magnetic resonance (C-NMR) spectrum of the product of Example 21;
FIG. 5 is a pipeline flow diagram of a 3-methoxypropionate production unit of a reaction system; and
FIG. 6 is a pipeline flow diagram of a 3-methoxy-N,N-dimethylpropionamide production unit of a reaction system.

### DETAILED DESCRIPTION

The technical solutions in the embodiments of the present application will be clearly and completely described below in combination with the examples of the application. Obviously, the described examples represent only a part of the embodiments of the present application, but not all of them. All other embodiments obtained by those skilled in the art based on the embodiments of the present application without creative work fall within the scope of protection of the present application.

### Reaction System

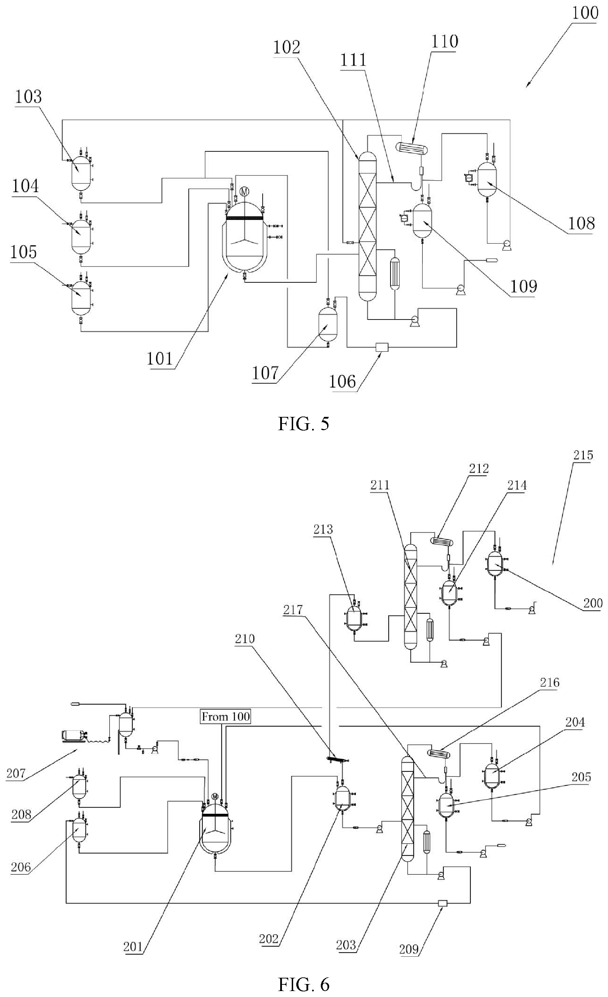

Referring to FIG. 5 and FIG. 6, a batch production system for 3-methoxy-N,N-dimethylpropionamide includes a 3-methoxypropionate production unit 100 and a 3-methoxy-N,N-dimethylpropionamide production unit 200;
the 3-methoxypropionate production unit 100 includes a first reactor 101 and a first rectification column 102 connected to the first reactor 101; the top of the first rectification column 102 is connected to a first light component collection subunit and a second light component collection subunit; the outlet of the second light component collection subunit is connected to the feed end of the 3-methoxy-N,N-dimethylpropionamide production unit 200; and
the outlet of the bottom of the first rectification column 102 is connected to the inlet of the first reactor 101.

Regarding this embodiment, the 3-methoxypropionate production unit 100 carries out the Michael addition reaction, and the 3-methoxy-N,N-dimethylpropionamide production unit 200 carries out the amidation reaction. The specific process flow is as follows:
The Michael addition reaction includes the following steps:
step 1: carrying out a Michael addition reaction using methyl acrylate and methanol as raw materials in the first reactor 101 in the presence of a basic catalyst such as sodium methoxide to obtain a first mixed solution containing methyl 3-methoxypropionate; and
step 2: removing methanol via the first rectification column 102, and collecting distillate fractions in a preset temperature range to obtain methyl 3-methoxypropionate.

The kettle residue only contains a small amount of white solid viscous material, mainly composed of sodium methoxide and the intermediate. It can be fed into the first reactor 101 for reuse in the next Michael addition reaction.

The specific process of the amidation reaction will be described later.

Preferably, the 3-methoxypropionate production unit 100 further includes a first raw material tank 103, a second raw material tank 104, and a third raw material tank 105. The outlet of the first raw material tank 103, the outlet of the second raw material tank 104, and the outlet of the third raw material tank 105 are respectively connected to the inlet of the first reactor 101. The outlet of the first light component collection subunit communicates with the first raw material tank 103.

The first raw material tank 103 is used for collecting methanol collected by the first rectification column 102. The second raw material tank 104 is used for supplying methyl acrylate. The third raw material tank 105 is used for supplying anhydrous methanol.

It is not recommended to directly reuse the kettle residue from the bottom of the first rectification column 102; it should be refined. Specifically, a first refining subunit is provided between the outlet of the bottom of the first rectification column 102 and the inlet of the first reactor 101.

Since the kettle residue is a white solid viscous material, it is preferable to dilute the kettle residue before refining to improve fluid mobility and avoid the catalyst being in solid form. Methanol can be used as the solvent, which can be introduced via pipeline as anhydrous methanol or using methanol from the first light component collection subunit.

More preferably, the outlet of the first light component collection subunit is connected to the column body of the first rectification column 102 via a pipeline, for injecting methanol into the first rectification column 102 during the later stage of rectification to dilute the kettle residue.

In this embodiment, the first refining subunit includes a first filter 106 and a first buffer tank 107 connected in sequence; the inlet of the first filter 106 communicates with the outlet of the bottom of the first rectification column 102; the outlet of the first buffer tank 107 communicates with the inlet of the first reactor 101. The inlet of the first buffer tank 107 is also connected to the outlet of the first raw material tank 103.

The kettle residue is filtered through the first filter 106, formulated in the first buffer tank 107. Methanol is introduced from the first raw material tank 103 to dilute the catalyst, and after dilution, it can be introduced into the first reactor 101 for reuse.

As a further refinement of this embodiment, the first light component collection subunit is a second buffer tank 108, and the second light component collection subunit is a third buffer tank 109; the second buffer tank 108 and the third buffer tank 109 are connected in parallel, and a first condenser 110 is connected between the inlets of the second buffer tank 108 and the third buffer tank 109 and the top of the first rectification column 102; a first reflux pipe 111 is provided at the outlet of the first condenser 110 and connected to the top of the first rectification column 102.

In some embodiments, two condensers can also be arranged at the top of the column, one connected to the second buffer tank 108 and one connected to the third buffer tank 109.

During production, reflux is required for both methanol collection and 3-methoxypropionate collection, which is common knowledge in the field and will not be overly limited here.

Next, the 3-methoxy-N,N-dimethylpropionamide production unit 200 is described in detail. Referring to FIG. 6, its pipeline layout is quite similar to that of the 3-methoxypropionate production unit 100. A major difference is the addition of a flash kettle 202. The reason is that in the amidation reaction, the reaction product composition is relatively complex, including not only the raw material (containing 3-methoxypropionate and methanol) sent from the Michael addition reaction but also dimethylamine, product, and glycerol. Therefore, methanol and dimethylamine are first removed by flash evaporation, and then the product, 3-methoxypropionate, and glycerol are separated in the second rectification column 203. It should be additionally noted that a flash kettle 202 can also be arranged between the first reactor 101 and the first rectification column 102 in the 3-methoxypropionate production unit 100.

Specifically, the 3-methoxy-N,N-dimethylpropionamide production unit 200 includes a second reactor 201, a flash kettle 202, and a second rectification column 203 connected in sequence; the outlet of the second light component collection subunit is connected to the inlet of the second reactor 201; the top of the second rectification column 203 is connected to an intermediate collection subunit 204 and a product collection subunit 205, both of which are storage tanks. The intermediate collection subunit 204 is used for collecting unreacted 3-methoxypropionate, and the product collection subunit 205 is used for collecting 3-methoxy-N,N-dimethylpropionamide.

The specific process of the amidation reaction is as follows:
step 3: synthesis: carrying out an amidation reaction using the distillate fraction obtained from the Michael addition reaction, a polyhydric alcohol, and dimethylamine as raw materials in the second reactor 201 to obtain a second mixed solution containing 3-methoxy-N,N-dimethylpropionamide;
step 4: performing a light component removal treatment on the second mixed solution in the flash kettle 202 to remove dimethylamine and methanol, so as to obtain a third mixed solution;
step 5: subjecting the third mixed solution to rectification with stepwise temperature increase under reduced pressure in the second rectification column 203, to obtain 3-methoxypropionate and 3-methoxy-N,N-dimethylpropionamide respectively in different temperature ranges, and a bottom is the polyhydric alcohol;
the 3-methoxypropionate and polyhydric alcohol obtained in the step 5 are recycled to the step 3.

Specifically, the 3-methoxy-N,N-dimethylpropionamide production unit 200 further includes a fourth raw material tank 206, a dimethylamine supply module 207, and a fifth raw material tank 208; the outlet of the fourth raw material tank 206, the outlet of the dimethylamine supply module 207, and the outlet of the fifth raw material tank 208 are connected to the inlet of the second reactor 201; the outlet of the bottom of the second rectification column 203 is connected to the inlet of the fourth raw material tank 206.

The fourth raw material tank 206 is used for storing the bottom liquid from the second rectification column 203, mainly glycerol.

The fifth raw material tank 208 is used for storing externally purchased glycerol.

Preferably, a second filter 209 is provided between the outlet of the bottom of the second rectification column 203 and the inlet of the fourth raw material tank 206.

The intermediate collection subunit 204 and the product collection subunit 205 are connected in parallel; a second condenser 216 is provided between the inlets of the intermediate collection subunit 204 and the product collection subunit 205 and the top of the second rectification column 203; a second reflux pipe 217 is provided at the outlet of the second condenser 216 and connected to the top of the second rectification column 203; the outlet of the intermediate collection subunit 204 is connected to the inlet of the second reactor 201.

From the detailed description of the process details above, it can be understood that the main component collected by the intermediate collection subunit 204 is the 3-methoxypropionate, along with a small amount of methanol.

As a further preference of this embodiment, the top of the flash kettle 202 is connected to a third condenser 210 and a third rectification column 211 in sequence, and preferably, a sixth buffer tank 213 is provided between the third condenser 210 and the third rectification column 211; the top of the third rectification column 211 is connected to a fourth condenser 212, and the outlet of the fourth condenser 212 is connected to a fourth buffer tank 214 and a fifth buffer tank 215 in parallel; the fourth buffer tank 214 is connected to the dimethylamine supply module 207, and the fifth buffer tank 215 is connected to the first raw material tank 103.

The fourth buffer tank 214 is used for collecting dimethylamine, and the fifth buffer tank 215 is used for collecting methanol, so as to achieve the reuse of dimethylamine and methanol.

It should be noted that: in this embodiment, valves, pumps, and other pipeline accessories or conveying power equipment are not described, and as is well known in the art, most pipelines in the chemical field are equipped with valves, and the type of valve can be freely selected according to the fluid properties; at some important or high-failure-rate valve positions, bypass valves should also be installed;
material transfer, reflux, etc., between containers, reactors, and columns are basically achieved using pumps and other operations in this field; except for when the requirement for material transfer speed is not strict, such as during material release, fluid flow can be achieved based on fluid gravity; in other places, pumps are generally used to achieve fluid transfer; there are many types and selections of pumps, such as diaphragm pumps, centrifugal pumps, peristaltic pumps, graphite pumps, etc.; in the present application, centrifugal pumps are mostly used.

Weighing modules are installed on most raw material tanks; thermometers and pressure gauges should be installed in reactors and columns; as is well known in the art, reactors should be equipped with necessary accessories such as heating and/or cooling jackets, stirring power devices, level gauges, etc., and if the reactor needs to operate under pressure or vacuum, it should be equipped with pressurizing pipes or vacuum pipes; generally, condensers are installed at the top of reactors, which can be horizontal condensers, vertical condensers, or a combination of both; rectification columns are conventionally designed in this field, the column bottom has a heat source, the column top has reflux, and rectification columns are generally packed columns; thermometers, pressure gauges, etc., are installed at different heights within the column, and if distillation under reduced pressure is required, the rectification column also has a vacuum pipe.

The above are all conventional designs and generally will not be described in detail again in the text description of the present application. In the drawings of the present application, some of the above accessories and equipment are labeled. If not labeled, it does not mean that the above accessories and equipment are not provided in the present application.

Declaration: 1. This reaction system is only used to explain the following reaction process and does not mean that the following reaction process must use the reaction system as described in this embodiment.

2. The following reaction processes are only small-scale laboratory tests. If they are to be applied to this reaction system, the amounts of raw materials in each reaction process below should be scaled up by a factor of 10 or more.

The following reaction processes have been verified in factory production, with high consistency between small-scale, pilot-scale, and large-scale tests.

### First Stage Reaction

Examples 1 to 18 and Comparative Examples 1 to 4 relate to the synthesis of the first-step intermediate.

### Example 1

At room temperature, 10.8 g of sodium methoxide (0.2 mol), 4 kg of methanol (125 mol), and 4.3 kg of methyl acrylate (50 mol) were added to the first reactor 101. The molar ratio of methyl acrylate:anhydrous methanol:sodium methoxide was 1:2.5:0.4%.

The mixed solution was stirred and reacted at 50°C for 2 hours. After the reaction, the appearance of the reaction mixed solution became clear and transparent. A sample was taken and quenched with concentrated sulfuric acid, followed by GC analysis. The methyl acrylate content was found to be below 1%, and the sum of other impurity contents was below 0.5%. Then, in the first rectification column 102, under a vacuum of -0.095 MPa, distillation under reduced pressure was performed. The reaction mixed solution was heated to 50°C, and methanol was collected (Fraction 1). As the distillate decreased, the temperature was gradually raised to 80°C, and the distillate (Fraction 1) was continuously collected until no more distillate was produced. A sample was taken for GC analysis. The temperature was then further raised to 90°C, and under a vacuum of -0.098 MPa, distillation under reduced pressure was performed, collecting distillate fractions from 68°C to 73°C (Fraction 2). As the distillate decreased, the temperature was gradually raised to 120°C to continue collecting distillate fractions (Fraction 2). A sample was taken for GC analysis. Finally, 5.72 kg of the intermediate methyl 3-methoxypropionate with a purity of 99.86% was obtained. The kettle residue only contained a small amount of white solid viscous material, mainly composed of sodium methoxide and the intermediate (the undistilled 3-methoxypropionate), which could be reused in the next batch.

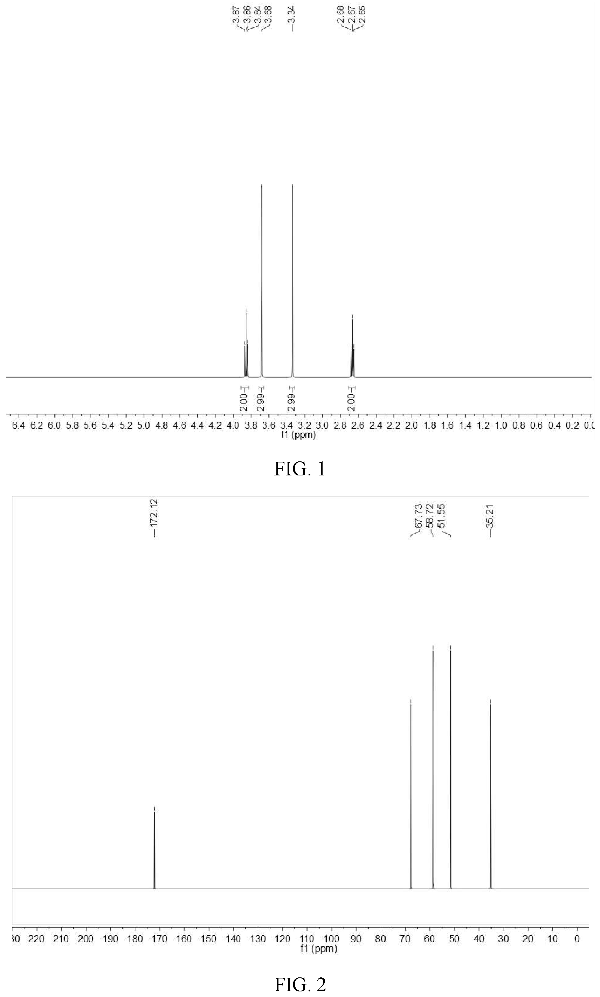

The NMR spectra of the intermediate methyl 3-methoxypropionate of the present application are shown in FIG. 1 and FIG. 2.

Here, several quantities and definitions appearing in the examples need to be explained, as follows:
I. In "1:2.5:0.4%", 0.4% means that the molar amount of sodium methoxide is only four thousandths of the molar amount of methyl acrylate.
II. Kettle residue: The residual component in the bottom of the rectification column.
III. Reuse: The kettle residue (sodium methoxide and intermediate) is discharged by some means (e.g., dilution) and, after necessary treatment (e.g., dilution, filtration), is used as a raw material for the reaction.

### Example 2

Compared with Example 1, the molar ratio of methyl acrylate:anhydrous methanol:sodium methoxide was changed from 1:2.5:0.4% to 1:2.5:0.6%, with other conditions remaining unchanged.

### Example 3

Compared with Example 1, the molar ratio of methyl acrylate:anhydrous methanol:sodium methoxide was changed from 1:2.5:0.4% to 1:2.5:0.3%, with other conditions remaining unchanged.

### Example 4

Compared with Example 1, the molar ratio of methyl acrylate:anhydrous methanol:sodium methoxide was changed from 1:2.5:0.4% to 1:2.5:0.2%, with other conditions remaining unchanged.

### Example 5

Compared with Example 1, the molar ratio of methyl acrylate:anhydrous methanol:sodium methoxide was changed from 1:2.5:0.4% to 1:1.5:0.4%, with other conditions remaining unchanged.

### Example 6

Compared with Example 2, the molar ratio of methyl acrylate:anhydrous methanol:sodium methoxide was changed from 1:2.5:0.4% to 1:2.0:0.4%, with other conditions remaining unchanged.

### Example 7

Compared with Example 1, the molar ratio of methyl acrylate:anhydrous methanol:sodium methoxide was changed from 1:2.5:0.4% to 1:3.0:0.4%, with other conditions remaining unchanged.

### Example 8

Compared with Example 4, the reaction temperature was changed from 50°C to 45°C, with other conditions remaining unchanged.

### Example 9

Compared with Example 4, the reaction temperature was changed from 50°C to 55°C, with other conditions remaining unchanged.

### Example 10

Compared with Example 4, the reaction temperature was changed from 50°C to 60°C, with other conditions remaining unchanged.

### Example 11

Compared with Example 4, the reaction time was increased from 2 hours to 3 hours, with other conditions remaining unchanged.

### Example 12

Compared with Example 4, the reaction time was increased from 2 hours to 5 hours, with other conditions remaining unchanged.

### Example 13

Compared with Example 1, the reaction time was shortened from 2 hours to 1 hour, with other conditions remaining unchanged.

### Example 14

Compared with Example 1, the reaction time was shortened from 2 hours to 0.5 hours, with other conditions remaining unchanged.

### Example 15

Compared with Example 1, the 10.8 g of sodium methoxide (0.2 mol) was changed to 4.8 g of sodium hydride (0.2 mol), with other conditions remaining unchanged.

### Example 16

Compared with Example 1, the 10.8 g of sodium methoxide (0.2 mol) was changed to 11.2 g of potassium hydroxide (0.2 mol), with other conditions remaining unchanged.

### Example 17

Compared to Example 1, the molar ratio of methyl acrylate:anhydrous methanol:sodium methoxide was changed from 1:2.5:0.4% to 1:2.5:0.1%, with other conditions remaining unchanged.

### Example 18

Compared to Example 1, the molar ratio of methyl acrylate:anhydrous methanol:sodium methoxide was changed from 1:2.5:0.4% to 1:1:0.4%, with other conditions remaining unchanged.

### Example 19

Compared with Example 1, the molar ratio of methyl acrylate:anhydrous methanol:sodium methoxide was changed from 1:2.5:0.4% to 1:3.2:0.4%, with other conditions remaining unchanged.

### Comparative Example 1

Compared with Example 1, the molar ratio of methyl acrylate:anhydrous methanol:sodium methoxide was changed from 1:2.5:0.4% to 1:2.5:1%, with other conditions remaining unchanged. During distillation under reduced pressure, it was found that the amount of the forecut fraction was abnormal and increased significantly. Sampling and GC analysis showed that the amount of methyl acrylate in the reaction mixed solution increased, the amount of the intermediate decreased, and a small amount of dimer of methyl acrylate was also detected.

### Comparative Example 2

Compared with Example 1, the molar ratio of methyl acrylate:anhydrous methanol:sodium methoxide was changed from 1:2.5:0.4% to 1:2.5:0.8%, with other conditions remaining unchanged.

### Comparative Example 3

Compared with Example 1, the molar ratio of methyl acrylate:anhydrous methanol:sodium methoxide was changed from 1:2.5:0.4% to 1:2.5:0.7%, with other conditions remaining unchanged.

### Comparative Example 4

Compared with Comparative Example 1, after the reaction, 0.18 kg of concentrated sulfuric acid was added to the reaction mixed solution for neutralization. The neutralized liquid was filtered using a conventional sand core funnel with a pore size of several tens of micrometers, which could only filter out a small amount of sulfate. The filtration was then changed to use a 0.45 µm filter membrane. Other conditions remained unchanged, and this distillation process proceeded normally.

The relevant reaction parameters and results of Examples 1-19 and Comparative Examples 1 to 4 are shown in Table 1 below;

**Table 1 Preparation Process Parameters and Results for Intermediate Methyl 3-Methoxypropionate in Examples 1 to 19 and Comparative Examples 1 to 4**

| | Feeding Molar Ratio (Methyl Acrylate: Methanol:Catalyst) | Methyl Acrylate Conversion Ratio /% | Reaction Temperature /°C | Reaction Time /h | Intermediate Yield /% | Intermediate Purity /% | Whether Acid Was Added After Reaction |
|---|---|---|---|---|---|---|---|
| Example 1 | 1:2.5:0.4 % | 99.15 | 50 | 2 | 96.56 | 99.86 | No |
| Example 2 | 1:2.5:0.6% | 99.26 | 50 | 2 | 96.86 | 99.77 | No |
| Example 3 | 1:2.5:0.3% | 87.93 | 50 | 2 | 85.75 | 99.60 | No |
| Example 4 | 1:2.5:0.2% | 83.17 | 50 | 2 | 81.93 | 99.37 | No |
| Example 5 | 1:1.5:0.4% | 68.11 | 50 | 2 | 66.64 | 97.71 | No |
| Example 6 | 1:2:0.4% | 82.49 | 50 | 2 | 80.29 | 98.53 | No |
| Example 7 | 1:3:0.4% | 99.00 | 50 | 2 | 94.95 | 99.68 | No |
| Example 8 | 1:2.5:0.2% | 74.74 | 45 | 2 | 69.42 | 97.44 | No |
| Example 9 | 1:2.5:0.2% | 80.72 | 55 | 2 | 76.80 | 98.86 | No |
| Example 10 | 1:2.5:0.2% | 78.57 | 60 | 2 | 71.92 | 98.51 | No |
| Example 11 | 1:2.5:0.2% | 90.16 | 50 | 3 | 87.34 | 99.12 | No |
| Example 12 | 1:2.5:0.2% | 95.56 | 50 | 5 | 93.16 | 99.56 | No |
| Example 13 | 1:2.5:0.6% | 99.01 | 50 | 1 | 95.86 | 99.68 | No |
| Example 14 | 1:2.5:0.6% | 98.56 | 50 | 0.5 | 95.24 | 99.71 | No |
| Example 15 | 1:2.5:0.4% | 99.08 | 50 | 2 | 96.34 | 99.78 | No |
| Example 16 | 1:2.5:0.4% | 97.86 | 50 | 2 | 95.28 | 99.57 | No |
| Example 17 | 1: 2.5: 0.1% | 56.12 | 50 | 2 | 52.86 | 98.23 | No |
| Example 18 | 1: 1: 0.4% | 38.91 | 50 | 2 | 35.02 | 97.35 | No |
| Example 19 | 1: 3.2: 0.4% | 98.89 | 50 | 2 | 94.12 | 98.95 | No |
| Comparative Example 1 | 1:2.5:1% | 99.21 | 50 | 2 | - | - | No |
| Comparative Example 2 | 1:2.5:0.8% | 99.26 | 50 | 2 | - | - | No |
| Comparative Example 3 | 1:2.5:0.7% | 99.16 | 50 | 2 | - | - | No |
| Comparative Example 4 | 1:2.5:1% | 99.31 | 50 | 2 | 94.54 | 99.69 | Yes |

From the results in Table 1, it was found that in Examples 1 to 2 and Comparative Examples 1 to 3, under the conditions of 50°C and reaction for 2 hours, the conversion ratio could reach 99% when using 0.4% to 1% catalyst, and there were essentially no by-products. From Examples 2, 11, and 12, it was found that when using 0.2% catalyst, extending the reaction time from 2 hours to 5 hours increased the conversion ratio from 83% to over 95%. From Examples 1, 13, and 14, it was found that when using 0.6% catalyst, shortening the reaction time from 2 hours to 0.5 hours could still achieve a conversion ratio of over 98%.

**Table 2 Distillation Results in Examples 1 to 2 and Comparative Examples 1 to 4**

| | Feeding Molar Ratio (Methyl Acrylate:Methanol:Catalyst) | Methyl Acrylate Content in Reaction Mixed Solution /% | Methyl Acrylate Content in Fraction 1 /% | Methyl Acrylate Content in Fraction 2 /% | Whether Acid Was Added for Neutralization After Reaction |
|---|---|---|---|---|---|
| Example 1 | 1:2.5:0.4 % | 0.41 | 1.53 | 0.14 | No |
| Example 2 | 1:2.5:0.6% | 0.39 | 1.52 | 0.22 | No |
| Comparative Example 1 | 1:2.5:1 % | 0.37 | 24.76 | 4.88 | No |
| Comparative Example 2 | 1:2.5:0.8% | 0.40 | 8.52 | 2.10 | No |
| Comparative Example 3 | 1:2.5:0.7% | 0.35 | 3.72 | 0.56 | No |
| Comparative Example 4 | 1:2.5:1% | 0.38 | 1.46 | 0.28 | Yes |

From Table 2, it was found that when the amount of sodium methoxide is ≥ 0.7%, without acid neutralization after the reaction, the methyl acrylate content increases during the heating and distillation process, and it raises as the amount of sodium methoxide increases. In Comparative Example 1, during the distillation process, the methyl acrylate content in Fraction 1 reached over 24%, and 1.49% of the dimer of methyl acrylate was detected in Fraction 2. Methyl acrylate polymer was detected at 16.23% in the kettle residue, indicating that under strong alkaline conditions, the intermediate decomposes at high temperature to generate methyl acrylate, which further undergoes polymerization reactions. In Comparative Example 4, compared with Comparative Example 1, acid neutralization was performed after the reaction. Although the distillation process proceeded normally, waste salts were generated. However, only a small amount of sulfate could be filtered out using a conventional sand core funnel with a pore size of several tens of micrometers, requiring filtration with a 0.45 µm filter membrane, which is difficult to separate industrially. Moreover, part of the product was adsorbed in the waste salts, causing the yield to decrease from 96% to 94%.

### Example 20

The kettle residue from Example 2 was reused, with other conditions remaining unchanged.

The reuse rules can be referred to in Table 3. In Table 3, "Methyl acrylate:Methanol = 1:2.5, Reusing kettle residue from Example 2" means: the raw material feeding molar ratio is methyl acrylate:methanol = 1:2.5; sodium methoxide is not additionally fed but entirely reused, maintaining methyl acrylate:sodium methoxide = 1:0.6%; the small amount of the intermediate 3-methoxypropionate in the kettle residue is negligible.

Examples 21 to 23 refer to this rule.

### Example 21

The kettle residue from Example 20 was reused, with other conditions remaining unchanged.

### Example 22

The kettle residue from Example 21 was reused, with other conditions remaining unchanged.

### Example 23

The kettle residue from Example 22 was reused, with other conditions remaining unchanged.

The reaction reuse results for Examples 21 to 23 can be referred to in Table 3.

**Table 3 Results of Reuse Examples for the First Step Reaction**

| | Feeding Molar Ratio | Methyl Acrylate Conversion Ratio /% | Reaction Temperature /°C | Reaction Time /h | Intermediate Yield/% | Intermediate Purity/% | Number of Reuses |
|---|---|---|---|---|---|---|---|
| Example 2 | Methyl Acrylate:Methanol:S odium Methoxide =1:2.5:0.6% | 99.26 | 50 | 2 | 96.86 | 99.77 | 0 |
| Example 20 | Methyl Acrylate:Methanol = 1:2.5, Reusing kettle residue from Example 2 | 99.25 | 50 | 2 | 98.32 | 99.66 | 1 |
| Example 21 | Methyl Acrylate:Methanol = 1:2.5, Reusing kettle residue from Example 17 | 99.12 | 50 | 2 | 98.16 | 99.72 | 2 |
| Example 22 | Methyl Acrylate:Methanol = 1:2.5, Reusing kettle residue from Example 18 | 90.06 | 50 | 2 | 97.98 | 99.85 | 3 |
| Example 23 | Methyl Acrylate:Methanol = 1:2.5, Reusing kettle residue from Example 19 | 98.85 | 50 | 2 | 97.55 | 99.69 | 4 |

From Example 20 in Table 3, it was found that after the kettle residue from rectification was reused once, the yield increased slightly. This is because there was still a small amount of methyl 3-methoxypropionate in the kettle residue. After the next reuse, under the same conversion ratio and yield of the reaction, the yield naturally increased. From Example 23, it can be seen that after 4 reuses, the yield of the intermediate basically did not change, with only a slight decrease compared to 3 reuses.

### Second Stage Reaction

Examples 24 to 36 relate to the synthesis of the second-step product.

### Example 24

At 0°C, 2.36 kg of the intermediate, 1.84 kg of glycerol, and 1.8 kg of dimethylamine were added to the second reactor 201. The molar ratio of the intermediate methyl 3-methoxypropionate:dimethylamine:glycerol was 1:2:1. The temperature was raised to 35°C, and the mixed solution was stirred and reacted for 5 hours. After the reaction, the reaction mixed solution was transferred to the flash kettle 202. Under a vacuum of -0.098 MPa and at 45°C, distillation under reduced pressure was performed to separate a mixed solution of dimethylamine and methanol, which was further separated into dimethylamine (light component 1) and methanol in the third rectification column 211. The remaining reaction mixed solution was transferred to the second rectification column 203. Under a vacuum of -0.098 MPa, the temperature was raised to 90°C, and distillate fractions in the temperature range from 65°C to 80°C were collected, mainly consisting of the intermediate methyl 3-methoxypropionate (light component 2). The temperature was then further raised to 110°C, and distillate fractions in the temperature range from 80°C to 95°C were collected, mainly consisting of the product 3-methoxy-N,N-dimethylpropionamide. The remaining kettle residue mainly consisted of glycerol, which could be recovered and reused.

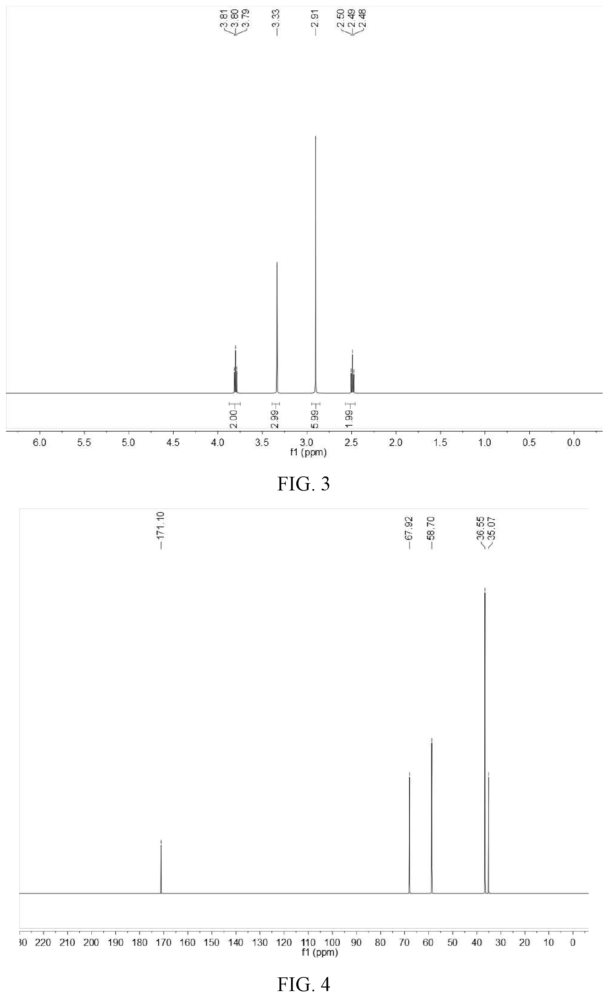

The NMR spectra of the product 3-methoxy-N,N-dimethylpropionamide obtained in this example are shown in FIG. 3 and FIG. 4.

### Example 25

Compared with Example 24, the reaction time was extended from 5 hours to 7 hours, with other conditions remaining unchanged.

### Example 26

Compared with Example 24, the reaction time was extended from 5 hours to 10 hours, with other conditions remaining unchanged.

### Example 27

Compared with Example 24, the reaction time was extended from 5 hours to 15 hours, with other conditions remaining unchanged.

### Example 28

Compared with Example 24, the molar ratio of the intermediate methyl 3-methoxypropionate:dimethylamine:glycerol was changed from 1:2:1 to 1:1.2:1.

### Example 29

Compared with Example 24, the molar ratio of the intermediate methyl 3-methoxypropionate:dimethylamine:glycerol was changed from 1:2:1 to 1:3:1.

### Example 30

Compared with Example 24, the molar ratio of the intermediate methyl 3-methoxypropionate:dimethylamine:glycerol was changed from 1:2:1 to 1:2:0.5.

### Example 31

Compared with Example 24, the molar ratio of the intermediate methyl 3-methoxypropionate:dimethylamine:glycerol was changed from 1:2:1 to 1:2:2.

### Example 32

Compared with Example 24, the reaction temperature was adjusted from 35°C to 30°C.

### Example 33

Compared with Example 24, the reaction temperature was adjusted from 35°C to 40°C.

### Example 34

Compared with Example 24, the reaction temperature was adjusted from 35°C to 50°C.

### Example 35

Compared with Example 24, the reaction temperature was adjusted from 35°C to 60°C.

### Example 36

Compared with Example 29, the reaction time was extended from 5 hours to 10 hours, with other conditions remaining unchanged.

Table 4 shows the reaction parameters and results for Examples 24 to 36.

**Table 4 Preparation Parameters and Results for Product 3-Methoxy-N,N-dimethylpropionamide in Examples 24 to 36**

| | Feeding Molar Ratio (Intermediate:Dimethylamine: Glycerol) | Intermediate Conversion Ratio/% | Reaction Temperature/°C | Reaction Time/h | Product Yield/% | Product Purity/% |
|---|---|---|---|---|---|---|
| Example 24 | 1:2:1 | 56.5 | 35 | 5 | 55.2 | 99.56 |
| Example 25 | 1:2:1 | 65.1 | 35 | 7 | 63.8 | 99.71 |
| Example 26 | 1:2:1 | 71.2 | 35 | 10 | 70.3 | 99.68 |
| Example 27 | 1:2:1 | 71.8 | 35 | 15 | 70.5 | 99.68 |
| Example 28 | 1:1.2:1 | 38.6 | 35 | 10 | 36.8 | 99.75 |
| Example 29 | 1:3:1 | 61.2 | 35 | 5 | 59.8 | 99.58 |
| Example 30 | 1:2:0.5 | 54.8 | 35 | 5 | 53.1 | 99.78 |
| Example 31 | 1:2:2 | 52.6 | 35 | 5 | 51.3 | 99.81 |
| Example 32 | 1:2:1 | 52.4 | 30 | 5 | 50.7 | 99.71 |
| Example 33 | 1:2:1 | 58.4 | 40 | 5 | 56.1 | 99.67 |
| Example 34 | 1:2:1 | 58.9 | 50 | 5 | 56.6 | 99.59 |
| Example 35 | 1:2:1 | 61.2 | 60 | 5 | 53.6 | 99.69 |
| Example 36 | 1:3:1 | 76.5 | 35 | 10 | 74.8 | 99.68 |

From Examples 24 to 27, 29, and 36, it can be seen that under catalyst-free conditions, extending the reaction time can increase the conversion ratio and yield. Under the conditions of Example 36 with a reaction time of 10 hours, the yield reached 74.8%. From Examples 24 and 32-34, it can be seen that when the reaction temperature is too low, the conversion ratio of the intermediate is too low, leading to a lower yield. However, when the temperature is too high, while the conversion ratio increases, side reactions become more numerous, selectivity worsens, and the yield actually decreases.

Examples 37 to 40 and Comparative Examples 5 to 7 are reuse experiments for the second-step reaction.

### Example 37

The recovered light component 2 and glycerol from Example 26 were reused. The reaction was carried out by feeding with a mass ratio of (intermediate + light component 2):glycerol:dimethylamine = 1.28:1:1. The feeding amount of the intermediate remained unchanged. Other conditions were kept the same as in Example 26.

### Example 38

The recovered light component 2 and glycerol from Example 37 were reused. Other conditions were kept the same as in Example 26.

### Example 39

The recovered light component 2 and glycerol from Example 38 were reused. Other conditions were kept the same as in Example 26.

### Example 40

The recovered light component 2 and glycerol from Example 39 were reused. Other conditions were kept the same as in Example 26.

### Comparative Example 5

Different from Example 26, a catalyst, sodium methoxide, was added. The molar ratio of intermediate methyl 3-methoxypropionate:dimethylamine:glycerol:sodium methoxide was 1:2:1:2 %. After low-temperature distillation to remove dimethylamine and methanol, concentrated sulfuric acid was added for neutralization, and the waste salts were filtered off. Other conditions were kept the same as in Example 26.

### Comparative Example 6

The recovered light component 2 and glycerol from Comparative Example 5 were reused. The reaction was carried out by feeding with a mass ratio of (intermediate + light component 2):glycerol:dimethylamine:sodium methoxide = 1.28:1:1:0.012. Other conditions were kept the same as in Comparative Example 5.

Table 5 shows the results of the reuse experiments for the second-step reaction.

**Table 5. Results of Reuse Experiments for the Second-Step Reaction**

| | Feeding Molar Ratio | Single-run Yield /% | Cumulative Yield from Reuse /% | Number of Reuses |
|---|---|---|---|---|
| Example 26 | Intermediate:Dimethylamine:Glycerol=1:2:1 | 70.3 | 70.3 | 0 |
| Example 37 | Intermediate:Dimethylamine:Glycerol=1:2:1 | 68.8 | 79.2 | 1 |
| Example 38 | Intermediate:Dimethylamine:Glycerol=1:2:1 | 69.1 | 84.9 | 2 |
| Example 39 | Intermediate:Dimethylamine:Glycerol=1:2:1 | 71.1 | 88.1 | 3 |
| Example 40 | Intermediate:Dimethylamine:Glycerol=1:2:1 | 68.5 | 90.6 | 4 |
| Comparativ e Example 5 | Intermediate:Dimethylamine:Glycerol:Sodiu m Methoxide=1:2:1:2 % | 90.3 | 90.3 | 0 |
| Comparativ e Example 6 | Intermediate:Dimethylamine:Glycerol:Sodiu m Methoxide=1:2:1:2 % | 38.2 | 64.7 | 1 |

### Result Analysis:

1. From Table 5, it can be seen that after reusing the raw materials for 4 cycles, the single-run yield remained largely unchanged, and the cumulative yield from reuse exceeded 90%, indicating a high recycling efficiency of the raw materials. In the comparative examples where sodium methoxide catalyst was added, although the yield from the first run was very high, the single-run yield after one reuse dropped to less than 40%. This is because a large amount of sulfate salts dissolved in the glycerol. After heating and distillation, the color of the glycerol darkened significantly, and its viscosity increased substantially, severely affecting the reaction progress and making it unsuitable for use as a solvent.
2. In the first-step reaction of the present application, by controlling the feeding amount of sodium methoxide, there is no need for acid neutralization after the reaction. Distillation under reduced pressure can be directly performed by heating to separate methanol and the intermediate product, without the occurrence of other side reactions. When the amount of sodium methoxide is excessive, during the distillation separation process, methyl 3-methoxypropionate undergoes decomposition under alkaline conditions, generating methyl acrylate and methanol. Methyl acrylate further undergoes polymerization reactions at high temperatures under alkaline conditions. When the amount of sodium methoxide is too low, the reaction conversion ratio is relatively low.

If basic catalysts such as sodium alkoxide are used in the second-step reaction, neutralization treatment is required after the reaction, producing a large amount of waste salts. These waste salts partially dissolve in methanol and glycerol. Simultaneously, methyl 3-methoxypropionate hydrolyzes under acidic conditions to generate 3-methoxypropionic acid. This method is neither environmentally friendly nor easy for separation, and it also generates a large number of by-products. Without acid neutralization treatment, when the temperature is too high, the product 3-methoxy-N,N-dimethylpropionamide and dimethylamine further react to generate the by-product 3-dimethylamino-N,N-dimethylpropionamide. Additionally, 3-methoxy-N,N-dimethylpropionamide decomposes to generate methyl 3-(dimethylamino)acrylate.

In the second-step reaction of the present application, no catalyst is used, eliminating the need for post-reaction catalyst separation and treatment. Using glycerol as a solvent, and by controlling the raw material feeding ratio and temperature, the conversion ratio after 10 hours of reaction can still reach over 70%. Moreover, side reactions are reduced, selectivity is improved, and the ratio of reaction yield to conversion ratio increases to above 0.98. In the post-treatment, high-temperature distillation to remove glycerol is not required. After distillation under reduced pressure to remove light components and the product, the main component in the kettle residue is glycerol, which also contains a small amount of product and trace amounts of the intermediate. This can be reused, avoiding issues related to glycerol's high boiling point and difficulty in recovery.

In summary, the technical advantages of the present application are:
1. After the synthesis of methyl 3-methoxypropionate, distillation operation is directly performed to separate methyl 3-methoxypropionate. By precisely controlling the amount of catalyst, dual objectives are achieved: the product does not decompose after distillation, and the catalyst can be retained. This approach retains the advantage of the traditional method of obtaining high-purity methyl 3-methoxypropionate after neutralization, and also avoids the challenges in the subsequent preparation of 3-methoxy-N,N-dimethylpropionamide, where the presence of catalyst would necessitate neutralization, and if not neutralized, the product would easily decompose during distillation.
2. By optimizing the amount of dimethylamine and temperature control, the present application achieves high conversion ratio and high selectivity under catalyst-free conditions.

Applicants state that the present application illustrates the process method through the aforementioned embodiments, but the present application is not limited to these process steps, nor does it mean that the present application must rely on these process steps to be implemented. Those skilled in the art should understand that any improvements to the present application, equivalent substitutions of the raw materials selected for the present application, addition of auxiliary components, selection of specific methods, etc., all fall within the protection scope and disclosure scope of the present application.

## Claims

1. A method for preparing a 3-methoxypropionate, wherein the method comprises the following steps:
step 1: carrying out a Michael addition reaction using an acrylate and methanol as raw materials in the presence of a basic catalyst to obtain a first mixed solution comprising methyl 3-methoxypropionate, wherein a molar ratio of the basic catalyst to the acrylate is less than or equal to 0.6:100; and
step 2: treating the first mixed solution by distillation, and collecting distillate fractions in a preset temperature range to obtain methyl 3-methoxypropionate;
a chemical formula of the acrylate is: 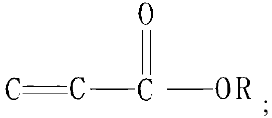 and
R is an alkyl group having 1 to 3 carbon atoms.

2. The method for preparing a 3-methoxypropionate according to claim 1, wherein the molar ratio of the basic catalyst to the acrylate ranges from 0.2:100 to 0.6:100.

3. The method for preparing a 3-methoxypropionate according to claim 1, wherein a molar amount of methanol is not less than 1.5 times a molar amount of the acrylate.

4. The method for preparing a 3-methoxypropionate according to claim 2, wherein a molar ratio of the acrylate to methanol ranges from 1:1.5 to 1:3.

5. The method for preparing a 3-methoxypropionate according to claim 1, wherein in the step 2, the first mixed solution is treated by distillation under reduced pressure, and the distillate fractions in the preset temperature range are collected to obtain methyl 3-methoxypropionate;
a pressure of the distillation ranges from -0.06 MPa to -0.1 MPa, preferably from -0.06 MPa to -0.095 MPa; and
the preset temperature range is 65°C to 120°C, preferably 68°C to 116°C.

6. The method for preparing a 3-methoxypropionate according to claim 5, wherein in the step 2, methanol is first removed by distillation under reduced pressure, then temperature is raised, and the distillate fractions in the preset temperature range are collected by distillation under reduced pressure.

7. The method for preparing a 3-methoxypropionate according to claim 6, wherein when the pressure of the distillation ranges from -0.09 MPa to -0.1 MPa, rectification is performed using a rectification column with stepwise temperature increase, methanol is distilled off by gradually increasing the temperature within a range of 50°C to 80°C; thereafter the temperature is raised to 90°C and then gradually increased to 120°C, and the distillate fractions at 68°C to 73°C are collected.

8. The method for preparing a 3-methoxypropionate according to claim 7, wherein a bottom product from the rectification column is a first heavy component comprising the basic catalyst and 3-methoxypropionate; the first heavy component is recycled to the step 1.

9. The method for preparing a 3-methoxypropionate according to any one of claims 1 to 8, wherein R is methyl, ethyl, or propyl.

10. The method for preparing a 3-methoxypropionate according to any one of claims 1 to 8, wherein the basic catalyst is one or a combination of an alkali metal alkoxide, an alkaline earth metal alkoxide, an alkali metal oxide, an alkaline earth metal oxide, an alkali metal hydroxide, an alkaline earth metal hydroxide, an alkali metal carbonate, an alkaline earth metal carbonate, a quaternary ammonium salt, and an organic base-type basic catalyst;
preferably, the basic catalyst is one or more of sodium methoxide, sodium ethoxide, sodium tert-butoxide, potassium tert-butoxide, sodium hydride, sodium amide, sodium hydroxide, potassium hydroxide, sodium oxide, potassium oxide, sodium carbonate, potassium carbonate, sodium bicarbonate, potassium bicarbonate, potassium fluoride, and choline.

11. The method for preparing a 3-methoxypropionate according to any one of claims 1 to 8, wherein a reaction temperature of the step 1 ranges from 40°C to 60°C, and a reaction time of the step 1 ranges from 1 hour to 5 hours.

12. A method for preparing 3-methoxy-N,N-dimethylpropionamide, wherein the method comprises the following step:
step 3: carrying out an amidation reaction using the distillate fractions in the preset temperature range according to any one of claims 1 to 11, a polyhydric alcohol, and dimethylamine as raw materials to obtain a second mixed solution comprising 3-methoxy-N,N-dimethylpropionamide.

13. The method for preparing 3-methoxy-N,N-dimethylpropionamide according to claim 12, wherein after the step 3, the method further comprises:
step 4: treating the second mixed solution to remove dimethylamine and methanol, so as to obtain a third mixed solution; and
step 5: subjecting the third mixed solution to rectification with stepwise temperature increase under reduced pressure to obtain a 3-methoxypropionate and 3-methoxy-N,N-dimethylpropionamide respectively in different temperature ranges, and a bottom product is the polyhydric alcohol;
preferably, the 3-methoxypropionate and the polyhydric alcohol obtained in the step 5 are recycled to the step 3.

14. The method for preparing 3-methoxy-N,N-dimethylpropionamide according to claim 12, wherein a reaction temperature of the step 3 is not more than 60°C, preferably not more than 50°C, and more preferably not more than 40°C.

15. The method for preparing 3-methoxy-N,N-dimethylpropionamide according to claim 14, wherein the reaction temperature of the step 3 ranges from 30°C to 50°C, preferably from 30°C to 40°C.

16. The method for preparing 3-methoxy-N,N-dimethylpropionamide according to claim 12, wherein a molar amount of the 3-methoxypropionate, a molar amount of dimethylamine, and a molar amount of the polyhydric alcohol are in a ratio ranging from 1:(1.2 to 3):(0.5 to 2), preferably, the molar amount of the 3-methoxypropionate, the molar amount of dimethylamine, and the molar amount of the polyhydric alcohol are in a ratio ranging from 1:(2 to 3):(0.5 to 2).

17. The method for preparing 3-methoxy-N,N-dimethylpropionamide according to claim 12, wherein a reaction time of the step 3 is not less than 4 hours, preferably, the reaction time of the step 3 ranges from 4 hours to 15 hours.

18. The method for preparing 3-methoxy-N,N-dimethylpropionamide according to claim 12, wherein the polyhydric alcohol is one or more of glycerol, ethylene glycol, 1,2-propanediol, 1,4-butanediol, and 1,6-hexanediol.
